# FASCICULE DE BREVET EUROPEEN

(11) **EP 1 933 900 B1**
(45) Date de publication et mention de la délivrance du brevet: **04.04.2012**
(21) Numéro de dépôt: 06820154.0
(22) Date de dépôt: 05.10.2006
(51) Int. Cl.: A61M 1/36

(54) **UNITE DE FILTRATION POUR L'ELIMINATION SELECTIVE D'UNE SUBSTANCE CIBLE**
FILTEREINHEIT ZUR SELEKTIVEN ELIMINATION EINER ZIELSUBSTANZ
FILTERING UNIT FOR SELECTIVELY ELIMINATING A TARGET SUBSTANCE

(30) Priorité: 10.10.2005 FR 0553069
(43) Date de publication de la demande: 25.06.2008
(73) Titulaire: Maco Pharma S.A., 59420 Mouvaux (FR)
(72) Inventeur: SUMIAN, Chryslian, F-59130 Lambersart (FR); GODARD, David, F-59200 Tourcoing (FR)
(74) Mandataire: Herrou, Nathalie
(86) Numéro de dépôt international: PCT/FR2006/002240
(87) Numéro de publication internationale: WO 2007/042644

(56) Documents cités:
- EP-A- 0 526 678
- EP-A- 1 382 361
- US-A- 5 639 376

## Description

L'invention est relative à une unité de filtration pour l'élimination sélective d'une substance cible présente dans un fluide biologique, ainsi qu'un système à poches comprenant une telle unité de filtration.

Elle s'applique typiquement au cas où la substance cible est un agent infectieux indésirable tel qu'un virus, protéine prion, bactérie ou parasite, ou une substance exogène utilisée dans un procédé d'inactivation des pathogènes d'un fluide biologique destiné à être transfusé à un patient.

Le prion est l'agent responsable des encéphalopathies subaiguës spongiformes transmissibles, notamment de la variante de la maladie de Creutzfeldt-Jakob chez l'homme. Des études récentes ont montré qu'il existe un risque probable de transmission du prion lors de transfusions sanguines.

Il apparaît donc nécessaire d'éliminer des produits sanguins destinés à être transfusés, le risque infectieux lié au prion, au même titre que les autres agents pathogènes.

Dans le document WO-2004/090102, il est décrit des particules adsorbantes sous forme de résine capable de lier de façon sélective les protéines prion présentes dans le sang. Ces particules adsorbantes sont placées dans des colonnes.

De même, il est maintenant reconnu que les substances tels que le bleu de méthylène ou des dérivés de psoralène utilisés lors de traitement d'inactivation des pathogènes du sang doivent être éliminés des produits sanguins avant leur transfusion à un patient.

A cet effet, le document WO-00/74806 décrit des dispositifs de filtration intégrant des particules adsorbantes telles que le charbon actif ou des résines à base de polystyrène pour éliminer des substances d'inactivation des pathogènes du sang. Ces dispositifs sont également arrangés sous forme de colonnes contenant les particules adsorbantes.

Le document EP-A-1 382 361 divulgue une unité de filtration selon le préambule de la revandication 1.

Il apparaît nécessaire d'améliorer ce type de dispositifs de filtration comprenant des particules adsorbantes, afin notamment de respecter les contraintes particulières de la filtration du sang, par exemple, en terme de relargage des particules dans le filtrat, de vitesse de filtration et/ou d'hémolyse des globules rouges. Il est également important de disposer de dispositifs de filtration pouvant facilement être industrialisés, sans perte des particules, et stérilisés.

L'invention vise donc à proposer une unité de filtration répondant à ces exigences et qui permet d'éliminer sensiblement une substance cible présente dans le fluide biologique en laissant substantiellement inchangée la composition du fluide biologique après la filtration.

A cet effet et selon un premier aspect, l'invention porte sur une unité de filtration pour l'élimination sélective d'une substance cible d'un fluide biologique tel que le sang ou un composant sanguin, du type comprenant une enveloppe extérieure munie d'au moins un orifice d'entrée et d'au moins un orifice de sortie entre lesquels le fluide à filtrer s'écoule suivant une direction, l'enveloppe renfermant un media filtrant comprenant, empilées d'amont en aval :
- une structure amont d'élimination d'au moins la substance cible, ladite structure comprenant un empilement de couches réalisées à base d'un matériau non-tissé présentant des pores de diamètre moyen D qui est agencé pour permettre l'écoulement du fluide au travers desdites couches selon la direction d'écoulement, ladite structure comprenant en outre, interposées entre au moins certaines des couches de la structure amont, des particules de diamètre moyen supérieur au diamètre D, lesdites particules présentant une affinité pour la substance cible ;
- une structure aval de rétention des particules qui est formée d'au moins une couche de matériau poreux, le diamètre moyen des pores dudit matériau étant inférieur ou égal au diamètre D.

Selon un deuxième aspect, l'invention porte sur un système à poches pour l'élimination sélective d'une substance cible d'un fluide biologique tel que le sang ou un composant sanguin, comprenant une poche de recueil du filtrat, ladite poche étant reliée, par l'intermédiaire d'une tubulure et au niveau d'un orifice d'entrée, à un orifice de sortie d'une unité de filtration selon le premier aspect de l'invention.

L'invention sera comprise grâce à la description qui suit en référence aux dessins annexés, illustrant divers modes de réalisation.
La figure 1 représente de façon schématique une vue en coupe d'une unité de filtration selon un mode de réalisation de l'invention.
Les figures 2 à 6 représentent de façon schématique différents modes de réalisation du média filtrant d'une unité de filtration selon l'invention.
La figure 7 représente de façon schématique deux couches de matériau non-tissé de la structure amont d'une unité de filtration selon l'invention, fixées entre elles par soudures discrètes.
La figure 8 représente de façon schématique un système à poches selon l'invention comprenant une unité de filtration.

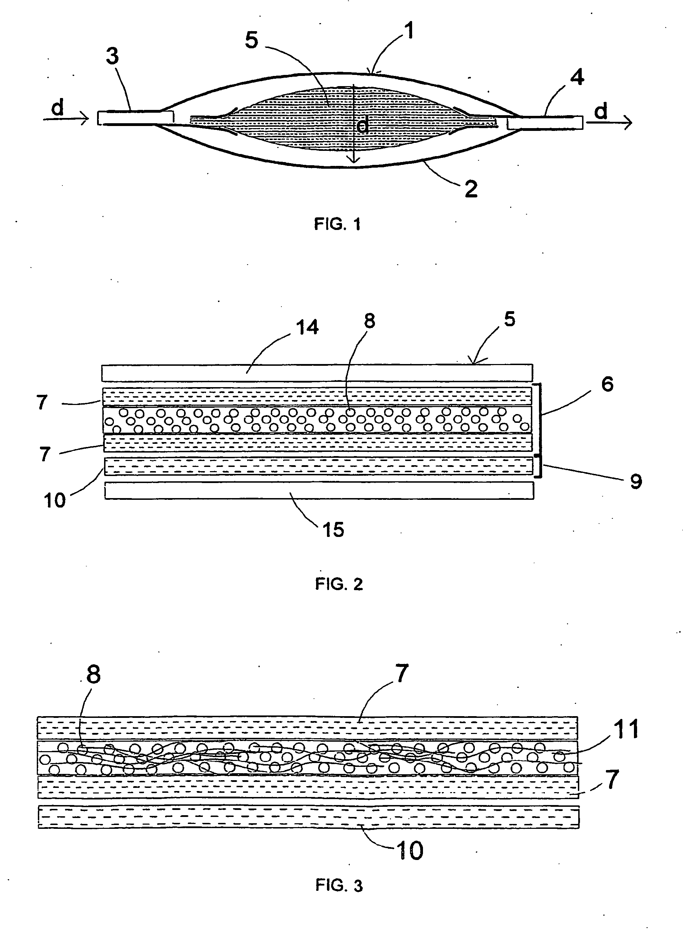

Dans la description qui suit, les termes "amont" et "aval" sont définis par rapport au sens d'écoulement du fluide dans l'unité de filtration, représenté par la flèche d de la figure 1.

En relation avec la figure 1, l'invention propose une unité de filtration 1 pour l'élimination sélective d'une substance cible d'un fluide biologique tel que le sang ou un composant sanguin.

Notamment, le fluide biologique est du sang total, un plasma, un sérum, un concentré de globules rouges, ou une suspension plaquettaire.

Par exemple, le fluide biologique comprend un sang total ou un concentré de globules rouges préalablement déleucocyté.

Selon un autre exemple, le fluide biologique est un sérum d'origine animale, utilisé dans des milieux de culture de cellules.

La substance cible à éliminer du fluide biologique est un agent pathogène ou infectieux tels qu'un virus, bactérie, parasite, champignon ou protéine prion. Lorsque le fluide biologique est destiné à être transfusé à un patient, il est nécessaire d'éliminer l'agent infectieux de ce fluide pour éviter de contaminer le patient.

Ceci est notamment vrai dans le cas de la protéine prion anormale (PrP^{sc}), responsable de la variante de la maladie de Creutzfeldt-Jakob chez l'homme.

En variante, la substance cible est une substance d'inactivation des pathogènes. De telles substances sont par exemple des agents photosensibles tels que les phénothiazines, notamment le bleu de méthylène, les dérivés de psoralène ou de porphyrine, ou des agents chimiques tels que les éthylèneimines. Ces substances peuvent être toxiques lorsqu'elles sont injectées à un patient et doivent aussi être éliminés du fluide biologique destiné à être transfusé.

L'élimination est totale ou partielle, c'est-à-dire suffisante pour supprimer ou réduire le risque infectieux lié aux agents infectieux, et/ou supprimer ou réduire la toxicité des substances d'inactivation, à un niveau acceptable pour la transfusion.

Selon la figure 1, l'unité de filtration 1 est du type comprenant une enveloppe extérieure 2 munie d'au moins un orifice d'entrée 3 et d'au moins un orifice de sortie 4 entre lesquels le fluide à filtrer s'écoule suivant une direction (d).

L'enveloppe 2 est souple, rigide ou semi-rigide. Notamment, lorsque l'enveloppe est souple, l'unité de filtration est du type décrit dans le document EP-A-0 526 678.

En relation avec la figure 2 et selon le premier aspect de l'invention, l'enveloppe renferme un media filtrant 5 comprenant, empilées d'amont en aval:
- une structure amont 6 d'élimination d'au moins la substance cible, ladite structure comprenant un empilement de couches 7 réalisées à base d'un matériau non-tissé présentant des pores de diamètre moyen D qui est agencé pour permettre l'écoulement du fluide au travers desdites couches selon la direction d'écoulement, ladite structure comprenant en outre, interposées entre au moins certaines des couches de la structure amont, des particules 8 de diamètre moyen supérieur au diamètre D, lesdites particules présentant une affinité pour la substance cible ;
- une structure aval 9 de rétention des particules qui est formée d'au moins une couche de matériau poreux 10, le diamètre moyen des pores dudit matériau étant inférieur ou égal au diamètre D.

La structure amont 6 permet l'élimination du fluide biologique d'au moins une substance cible, notamment la protéine prion anormale, tout en laissant passer les autres composants du fluide biologique.

Lorsque le fluide biologique est le sang ou un composant du sang, l'empilement de couches 7 d'un matériau non-tissé forme un filtre dit en profondeur, particulièrement adapté puisqu'il permet de laisser s'écouler les composants du sang comme les globules rouges, avec un risque réduit de colmatage ou blocage du média filtrant.

En particulier, les couches 7 de matériau non-tissé ont un diamètre moyen D supérieur ou égal à 8 µm. Cette porosité moyenne est adaptée au cas où le fluide biologique comprend des globules rouges.

Les couches 7 de la structure amont 6 ont des diamètres moyens de pores identiques ou différents. Avantageusement, les couches de la structure amont 6 forment un gradient de diamètres de pores décroissants dans le sens d'écoulement du fluide, de sorte à éviter le colmatage de l'unité de filtration 1.

L'élimination de la substance cible est réalisée au moins partiellement par l'intermédiaire des particules 8 qui sont capables de se lier par affinité à au moins un agent infectieux tel qu'un virus, bactérie, parasite, champignon et protéine prion, et/ou à une substance d'inactivation exogène.

Selon une variante, les particules 8 ont une affinité pour plus d'une substance cible, permettant ainsi l'élimination simultanée de plusieurs substances cibles d'un fluide biologique.

Les particules sont notamment des particules adsorbantes, telles que des particules de charbon actif, d'oxyde d'aluminium, de silice, ou à base de polymère comme le polystyrène ou le polyméthacrylate de méthyle.

Avantageusement, ces particules 8 sont traitées physiquement et/ou chimiquement pour améliorer leur spécificité et/ou leur affinité pour la ou les substance(s) cible(s).

Selon une réalisation particulière, les couches 7 de la structure amont 6 sont agencées pour éliminer par adsorption et/ou par filtration une partie de la substance cible.

En effet, selon les caractéristiques physico-chimiques des couches 7 de matériau non-tissé et leur arrangement dans l'unité de filtration 1, la substance cible est filtrée et/ou adsorbée partiellement par les couches 7 de la structure amont 6, augmentant ainsi la capacité de rétention de la substance cible de l'unité de filtration par rapport à la capacité de rétention des particules 8 seules, telles que arrangées dans une colonne.

Cette élimination par adsorption et/ou filtration s'ajoute à l'élimination de la substance cible par liaison aux particules 8.

Par exemple, des études récentes ont montré que les filtres à déleucocyter sont capables d'éliminer une partie des protéines prions présentes dans le sang ou le composant sanguin (Luisa Gregori, Lancet, vol. 364, Août 2004).

Selon une autre réalisation, les couches 7 de la structure amont 6 sont agencées pour éliminer par adsorption et/ou par filtration au moins un autre composant du fluide biologique.

Par exemple, lorsque les couches 7 de la structure amont 6 sont identiques ou similaires à celles utilisées dans un filtre à déleucocyter, l'unité de filtration 1 permet d'éliminer à la fois la substance cible et les leucocytes du fluide biologique. Une seule étape de filtration est alors nécessaire pour éliminer.deux substances indésirables d'un fluide biologique destiné à être réinjecté à un patient.

Selon la figure 2, les particules 8 sont interposées entre au moins certaines des couches 7 de la structure amont 6. C'est-à-dire, la structure amont comprend au moins deux couches 7 de matériau non-tissé entre lesquelles sont interposées les particules 8.

Selon un premier mode de réalisation, les particules 8 sont disposées entre les couches 7, c'est-à-dire qu'elles ne sont pas immobilisées sur les fibres des couches 7.

Par exemple, lors de la fabrication de l'unité de filtration, une première couche de matériau non-tissé est recouverte de particules, à l'aide d'un distributeur de particule, puis une deuxième couche de matériau non-tissé est empilée sur la première couche recouverte des particules, de sorte à former un sandwich comprenant des particules.

Le terme "sandwich" désigne deux couches 7 de matériau non-tissé de la structure amont 6, entre lesquelles sont disposées les particules 8.

Ce procédé de fabrication est facilement mis en oeuvre puisqu'il ne nécessite aucun traitement particulier des particules et/ou des couches pour lier ou adhérer les particules aux fibres des couches.

Selon un deuxième mode de réalisation représenté sur la figure 3, les particules 8 sont fixées à au moins une couche 11 réalisée en matériau non-tissé. La fixation est généralement réalisée par fonctionnalisation des particules et/ou des fibres, afin d'immobiliser les particules sur les fibres constituant la couche 11.

Dans ce cas, la couche 11 de fibres/particules est interposée entre deux couches 7 de matériau de non-tissé pour former le sandwich comprenant des particules intégrées dans un réseau fibreux.

Les particules 8 ont un diamètre moyen supérieur au diamètre moyen des couches 7 de matériau non-tissé formant la structure amont. Selon une réalisation particulière, les particules 8 sont formées de billes de résine de diamètre compris entre 20 et 150 µm.

Si l'unité de filtration 1 est destinée à éliminer les protéines prions d'un fluide biologique, les particules 8 sont, par exemple, celles décrites dans le document WO-2004/090102 et disponibles sous la référence commerciale Toyopearl™ Amino 650M.

La quantité totale de particules 8 présentes dans l'unité de filtration dépend de la capacité de rétention des particules 8 et des couches 7.

Selon une réalisation particulière, la quantité de particules entre deux couches est comprise entre 4 et 10 mg/cm². Ainsi, les particules restent suffisamment dispersées entre les deux couches pour laisser passer le fluide biologique.

En effet, une trop grande quantité de particules réduit la porosité totale du sandwich, pouvant ralentir l'écoulement du fluide biologique dans l'unité de filtration.

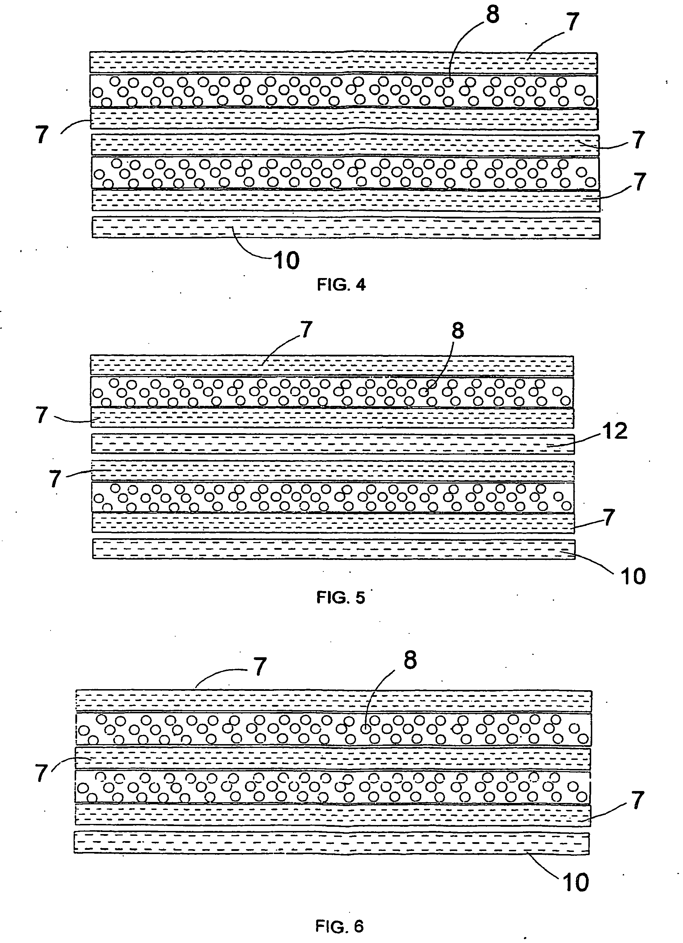

Comme représenté sur la figure 4, pour augmenter la quantité de particules 8 dans l'unité de filtration sans augmenter la quantité de particules entre deux couches 7, on augmente le nombre de sandwiches, dans l'unité de filtration. Selon une réalisation, le nombre de sandwiches dans l'unité de filtration est compris entre 2 et 20, notamment 6.

En relation avec la figure 5, on intercale une ou plusieurs couches de matériau poreux 12 entre les couches 7 formant les sandwiches.

En contrôlant des caractéristiques des couches intercalaires 12, comme leur nature ou leur porosité, il est possible notamment de réguler la vitesse d'écoulement du fluide dans l'unité de filtration 1 et donc le temps de contact entre le fluide et les particules 8.

Dans une réalisation particulière, ces couches intercalaires 12 ont un diamètre moyen de pores supérieur à la porosité des sandwiches comprenant les particules 8.

Ces couches intercalaires de matériau poreux 12 jouent alors le rôle de répartiteur de flux et facilitent l'écoulement du fluide dans l'unité de filtration, c'est-à-dire elles évitent une baisse de la vitesse d'écoulement du fluide dans l'unité de filtration due à l'augmentation du nombre de couches 7 et de la quantité de particules 8.

Par exemple, si le sandwich est composé de couches 7 de matériau poreux non-tissé de diamètre moyen d'environ 12 µm, la porosité de la couche intercalaire 12 est d'environ 12 µm.

En variante de cet exemple, les couches intercalaires sont réalisées en matériau thermoplastique tissé ou moulé, notamment sous forme de grille.

Selon une autre réalisation, la ou les couches intercalaires 12 ont un diamètre de pores inférieur ou égal à la porosité des sandwiches comprenant les particules 8.

Dans ce cas, les couches intercalaires ralentissent le flux de fluide dans l'unité de filtration, ce qui implique un temps de contact plus élevé entre le fluide biologique et les particules.

Selon une troisième réalisation, la ou les couches intercalaires 12 sont arrangées pour éliminer un autre composant sanguin du fluide biologique, par exemple les leucocytes.

L'augmentation du nombre de couches 7 peut cependant entraîner des difficultés de fabrication lors de l'incorporation de ces couches dans l'enveloppe extérieure de l'unité de filtration.

Pour résoudre cette difficulté et réduire le nombre de couches 7 dans l'unité de filtration tout en conservant la même quantité de particules 8 entre les couches 7, il est prévu comme représenté sur la figure 6, d'alterner des couches de matériau poreux 7 avec la couche de particules 8.

Ainsi, le nombre total de couches 7 est réduit par rapport à un empilement de sandwiches, ce qui facilite la fabrication de l'unité de filtration 1.

Il est également avantageux pour une meilleure élimination de la substance cible de répartir de façon homogène les particules 8 entres les couches de matériau non-tissé 7.

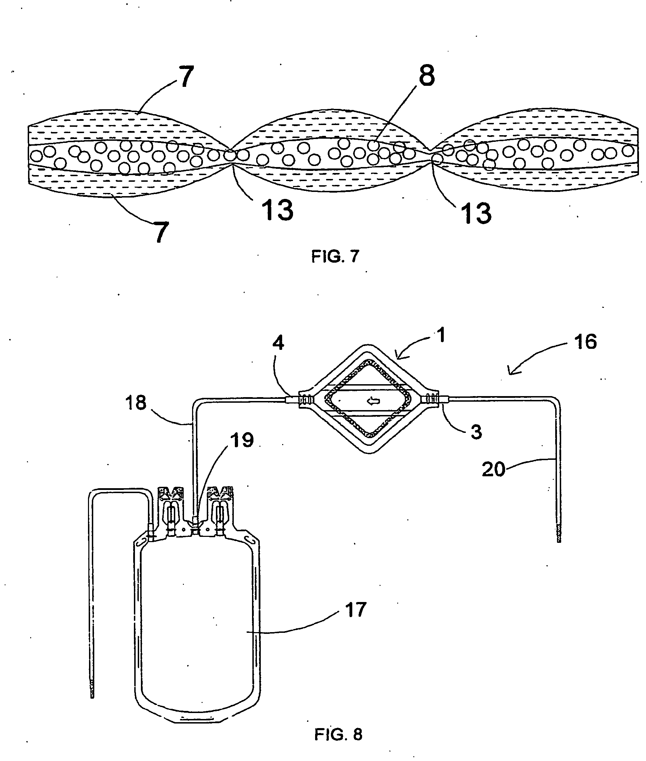

Pour conserver cette répartition homogène lors de la fabrication et l'utilisation de l'unité de filtration 1, et comme illustré sur la figure 7, au moins deux couches 7 sont soudées de façon discrète entre elles, de sorte à retenir les particules entre lesdites couches.

Cette façon de souder permet de maintenir fixées entre elles les deux couches 7 intercalant les particules 8.

Par exemple, les deux couches 7 entre lesquelles sont interposées les particules sont soudées par chaleur ou ultrasons à l'aide d'une calandre qui presse à chaud les couches au niveau de points séparés à intervalles réguliers les uns des autres, de sorte à créer des joints de soudure discrets 13.

Ce type de soudure par gaufrage laisse la porosité des couches 7 de matériau non-tissé inchangée entre les points de soudure 13.

Selon un mode réalisation, les périphéries d'au moins deux couches 7 sont soudées entre elles, les particules 8 étant enfermées entre lesdites couches.

Notamment, les périphéries des couches 7 de la structure amont 6 sont soudées par exemple par ultrasons. Cette construction évite la perte de particules lors de la fabrication de l'unité de filtration 1, notamment lors de la manipulation des différentes couches pour les empiler.

Selon une variante, les différentes couches 7 formant la structure amont sont coupées et soudées en une seule étape à l'aide d'un appareil de soudure et coupure par ultrasons.

Selon l'invention et comme illustré sur la figure 2, l'unité de filtration 1 comporte en aval de la structure amont 6, une structure aval 9 de rétention des particules 8 formée d'au moins une couche 10 de matériau poreux.

Cette couche 10 de matériau poreux possède un diamètre moyen de pores inférieur ou égal au diamètre D, notamment strictement inférieur au diamètre D.

Cette structure aval 9 possède au moins deux fonctions : la première est de prévenir toute fuite de particules de l'unité de filtration.

En effet, comme les couches 7 encadrant les particules 8 sont réalisées en matériau non-tissé, le diamètre des pores de ces couches 7 varie sur une plage. Il arrive alors que des particules 8 passent au travers des plus grands pores des couches 7 de matériau non-tissé.

Par exemple, la taille des particules 8 ayant une affinité pour la protéine prion varie entre 20 et 150 µm, notamment 40-90 µm avec un diamètre moyen de 65 µm ou 40-140 µm avec un diamètre moyen de 90 µm. Les couches 7 entre lesquelles sont interposées les particules 8 ont un diamètre moyen de 12 µm et varie entre 7 µm et 40 µm. Ainsi, il est possible que certaines petites particules 8 passent au travers des pores les plus grands de la couche 7 de non-tissé, provoquant le passage des particules 8 dans le fluide biologique filtré.

Dans le cas où le fluide biologique filtré doit être réinjecté à un patient, il est nécessaire d'éviter le passage de particules dans le filtrat. C'est la structure aval 9 qui réduit le risque de passage des particules dans le filtrat.

La deuxième fonction de la couche aval est de réguler le flux du fluide à la sortie de l'unité de filtration 1.

Par exemple, le matériau poreux 10 de la structure aval 9 de rétention est de type non-tissé.

D'autres exemples de type de matériau poreux utiles pour la structure aval sont les membranes, les matériaux tissés ou les grilles.

La porosité de la structure aval 9 dépend non seulement de la taille des particules 8, mais aussi par exemple, de sa nature et de la porosité des couches 7 de la structure amont 6.

En particulier, si les couches 7 de la structure amont 6 encadrant les particules 8 ont un diamètre moyen de pores supérieur à 8 µm, le diamètre moyen de pores de la couche 10 de matériau poreux de la structure aval 9 est inférieur ou égal à 8 µm.

Notamment, le diamètre moyen des pores de la couche de la structure aval 9 de rétention est inférieur à 10 µm.

Comme illustrée sur la figure 2, l'unité de filtration 1 comprend un média filtrant, qui, outre les structures amont et aval 6,9, comprend un pré et/ou un postfiltre 14,15.

Ces pré et/ou post filtre 14,15 sont identiques ou différents et réalisés par exemple en matériau poreux non-tissé.

Les matériaux formant les couches de média filtrant 5 sont choisis par exemple dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés.

Ces produits polymériques ne sont généralement pas hydrophiles naturellement et doivent être traités par des méthodes physiques et/ou chimiques, pour leur conférer lesdites propriétés hydrophiles nécessaires à la filtration du sang ou des composants sanguins.

De tels polymères rendus hydrophiles par traitement physique et/ou chimique sont disponibles sur le marché.

Selon un deuxième aspect de l'invention, on décrit ci-dessous un système à poches 16 pour l'élimination sélective d'une substance cible d'un fluide biologique tel que le sang ou un composant sanguin.

Selon la figure 8, le système à poches comprend une poche de recueil du filtrat 17, reliée par l'intermédiaire d'une tubulure 18 et au niveau d'un orifice d'entrée 19 à un orifice de sortie 4 d'une unité de filtration 1 telle que décrite ci-dessus.

En outre le système à poche comprend une tubulure 20 reliée à l'orifice d'entrée de l'unité de filtration 1.

En utilisation, une poche contenant le fluide à filtrer est reliée à la tubulure 20, par exemple à l'aide d'une connexion stérile. Puis, on laisse s'écouler le fluide à filtrer par gravité au travers de l'unité de filtration 1 et on recueille le fluide filtrer exempt de la substance cible dans la poche de recueil du filtrat 17.

Selon un autre mode de réalisation, l'unité de filtration est intégrée dans un système à poches dit clos, permettant le prélèvement du sang total et son traitement subséquent pour éliminer la substance cible du sang total ou d'un de ses composants.

## Revendications

1. Unité de filtration (1) pour l'élimination sélective d'une substance cible d'un fluide biologique tel que le sang ou un composant sanguin, du type comprenant une enveloppe extérieure (2) munie d'au moins un orifice d'entrée (3) et d'au moins un orifice de sortie (4) entre lesquels le fluide à filtrer s'écoule suivant une direction d, l'enveloppe (2) renfermant un média filtrant (5) **caractérisée en ce que** le média filtrant comprend, empilées d'amont en aval:
- une structure amont (6) d'élimination d'au moins la substance cible, ladite structure comprenant un empilement de couches (7) réalisées à base d'un matériau non-tissé présentant des pores de diamètre moyen D qui est agencé pour permettre l'écoulement du fluide au travers desdites couches (7) selon la direction d'écoulement (d), ladite structure (6) comprenant en outre, interposées entre au moins certaines des couches (7) de la structure amont, des particules (8) de diamètre moyen supérieur au diamètre D, lesdites particules (8) présentant une affinité pour la substance cible ;
- une structure aval (9) de rétention des particules (8) qui est formée d'au moins une couche (10) de matériau poreux, le diamètre moyen des pores dudit matériau étant inférieur ou égal au diamètre D.

2. Unité de filtration selon la revendication 1, **caractérisée en ce que** le diamètre moyen D est supérieur ou égal à 8 µm.

3. Unité de filtration selon la revendication 1 ou 2, **caractérisée en ce que** le diamètre moyen des pores de la couche (10) de la structure aval (9) est strictement inférieur au diamètre D.

4. Unité de filtration selon l'une quelconque des revendications 1 à 3, **caractérisée en ce que** les couches (7) de la structure amont sont agencées pour éliminer par adsorption et/ou par filtration une partie de la substance cible et/ou au moins un autre composant du fluide biologique.

5. Unité de filtration selon l'une quelconque des revendications 1 à 4, **caractérisée en ce que**.les particules (8) sont capables de se lier par affinité à au moins un agent infectieux tel qu'un virus, bactérie, parasite, champignon et protéine prion, et/ou à une substance d'inactivation exogène.

6. Unité de filtration selon l'une quelconque des revendications 1 à 5, **caractérisée en ce que** les particules (8) sont disposées entre les couches (7).

7. Unité de filtration selon l'une quelconque des revendications 1 à 5, **caractérisée en ce que** les particules (8) sont fixées à au moins une couche (11).

8. Unité de filtration selon l'une quelconque des revendications 1 à 7, **caractérisée en ce que** les particules (8) sont formées de billes de résine de diamètre compris entre 20 et 150 µm.

9. Unité de filtration selon l'une quelconque des revendications 1 à 8, **caractérisée en ce que** la quantité de particules (8) entre deux couches (7) est comprise entre 4 et 10 mg/cm².

10. Unité de filtration selon l'une quelconque des revendications 1 à 9, **caractérisée en ce qu'**au moins deux couches (7) sont soudées de façon discrète entre elles, de sorte à retenir les particules (8) entre lesdites couches (7).

11. Unité de filtration selon l'une quelconque des revendications 1 à 10, **caractérisé en ce que** les périphéries d'au moins deux couches (7) sont soudées entre elles, les particules (8) étant enfermées entre lesdites couches (7).

12. Unité de filtration selon l'une quelconque des revendications 1 à 11, **caractérisée en ce que** le matériau poreux de la structure aval (9) de rétention est de type non-tissé.

13. Unité de filtration l'une quelconque des revendications 1 à 12, **caractérisée en ce que** le diamètre moyen des pores de la couche (10) de la structure aval (9) de rétention est inférieur à 10 µm.

14. Unité de filtration selon l'une quelconque des revendications 1 à 13, **caractérisée en ce que** le media filtrant.(5) comprend en outre un pré et/ou un postfiltre (14,15).

15. Système à poches (16) pour l'élimination sélective d'une substance cible d'un fluide biologique tel que le sang ou un composant sanguin, **caractérisé en ce qu'**il comprend une poche de recueil du filtrat (17), ladite poche étant reliée, par l'intermédiaire d'une tubulure (18) et au niveau d'un orifice d'entrée (19), à un orifice de sortie (4) d'une unité de filtration (1) selon l'une quelconque des revendications 1 à 14.

## Claims

1. A filtration unit (1) for the selective elimination of a target substance from a biological fluid, such as blood or a blood component, of the type comprising an outer envelope (2) equipped with at least one inlet orifice (3) and at least one outlet orifice (4), between which the fluid to be filtered flows in a direction d, the envelope (2) enclosing a filtering medium (5), **characterised in that** the filtering medium comprises, stacked from upstream to downstream:
- an upstream structure (6) for eliminating at least the target substance, said structure comprising a stacking of layers (7) formed on the basis of a non-woven material having pores of mean diameter D which is arranged to allow the flow of the fluid through said layers (7) in the direction of flow d, said structure (6) further comprising, arranged between at least some of the layers (7) of the upstream structure, particles (8) of mean diameter greater than the diameter D, said particles (8) demonstrating an affinity for the target substance;
- a downstream structure (9) for retaining particles (8) which is formed of at least one layer (10) of porous material, the mean diameter of the pores of said material being less than or equal to the diameter D.

2. The filtration unit according to claim 1, **characterised in that** the mean diameter D is greater than or equal to 8 µm.

3. The filtration unit according to either claim 1 or 2, **characterised in that** the mean diameter of the pores of the layer (10) of the downstream structure (9) is strictly less than the diameter D.

4. The filtration unit according to any one of claims 1 to 3, **characterised in that** the layers (7) of the upstream structure are arranged to eliminate, by absorption and/or by filtration, some of the target substance and/or at least one other component of the biological fluid.

5. The filtration unit according to any one of claims 1 to 4, **characterised in that** the particles (8) are able to bond by affinity to at least one infectious agent, such as a virus, bacterium, parasite, fungus and prion protein, and/or to an exogenous inactivation substance.

6. The filtration unit according to any one of claims 1 to 5, **characterised in that** the particles (8) are disposed between the layers (7).

7. The filtration unit according to any one of claims 1 to 5, **characterised in that** the particles (8) are fixed to at least one layer (11).

8. The filtration unit according to any one of claims 1 to 7, **characterised in that** the particles (8) are formed of resin beads with a diameter between 20 and 150 µm.

9. The filtration unit according to any one of claims 1 to 8, **characterised in that** the amount of particles (8) between two layers (7) is between 4 and 10 mg/cm².

10. The filtration unit according to any one of claims 1 to 9, **characterised in that** at least two layers (7) are welded together discretely so as to retain the particles (8) between said layers (7).

11. The filtration unit according to any one of claims 1 to 10, **characterised in that** the peripheries of at least two layers (7) are welded together, the particles (8) being enclosed between said layers (7).

12. The filtration unit according to any one of claims 1 to 11, **characterised in that** the porous material of the downstream retaining structure (9) is of the non-woven type.

13. The filtration unit according to any one of claims 1 to 12, **characterised in that** the mean diameter of the pores of the layer (10) of the downstream retaining structure (9) is less than 10 µm.

14. The filtration unit according to any one of claims 1 to 13, **characterised in that** the filtering medium (5) further comprises a pre-filter and/or a post-filter (14,15).

15. A bag system (16) for the selective elimination of a target substance of a biological fluid, such as blood or a blood component, **characterised in that** it comprises a bag for collecting the filtrate (17), said bag being connected, via a tube (18) and at an inlet orifice (19), to an outlet orifice (4) of a filtration unit (1) according to any one of claims 1 to 14.

## Patentansprüche

1. Filtrationseinheit (1) zum selektiven Entfernen einer Zielsubstanz aus einem biologischen Fluid, wie etwa Blut, oder eines Blutbestandteils, des Typs, der eine äußere Hülle (2) umfasst, die mit mindestens einer Einlassöffnung (3) und mindestens einer Auslassöffnung (4) versehen ist, zwischen denen das zu filtrierende Fluid gemäß einer Richtung d strömt, wobei die Hülle (2) ein Filtermedium (5) beinhaltet, das **dadurch gekennzeichnet ist, dass** das Filtermedium, von stromaufwärtiger nach stromabwärtiger Richtung gestapelt, Folgendes umfasst:
- eine stromaufwärtige Struktur (6) zum Entfernen mindestens der Zielsubstanz, wobei die Struktur einen Stapel von Schichten (7) umfasst, ausgeführt auf der Basis eines Vliesmaterials, das Poren mit einem mittleren Durchmesser D aufweist, das gestaltet ist, um das Strömen des Fluids durch die Schichten (7) gemäß der Strömungsrichtung d zu ermöglichen, wobei die Struktur (6) ferner, eingebracht zwischen mindestens einige der Schichten (7) der stromaufwärtigen Struktur, Partikel (8) umfasst, die einen mittleren Durchmesser aufweisen, der größer ist als der Durchmesser D, wobei die Partikel (8) eine Affinität für die Zielsubstanz aufweisen;
- eine stromabwärtige Struktur (9) zum Zurückhalten der Partikel (8), die aus mindestens einer Schicht (10) aus porösem Material gebildet wird, wobei der mittlere Porendurchmesser des Materials kleiner oder gleich dem Durchmesser D ist.

2. Filtrationseinheit nach Anspruch 1, **dadurch gekennzeichnet, dass** der mittlere Durchmesser D größer oder gleich 8 µm ist.

3. Filtrationseinheit nach Anspruch 1 oder 2, **dadurch gekennzeichnet, dass** der mittlere Porendurchmesser der Schicht (10) der stromabwärtigen Struktur (9) exakt kleiner als der Durchmesser D ist.

4. Filtrationseinheit nach einem der Ansprüche 1 bis 3, **dadurch gekennzeichnet, dass** die Schichten (7) der stromaufwärtigen Struktur gestaltet sind, um durch Absorption und/oder durch Filtration einen Teil der Zielsubstanz und/oder mindestens eines anderen Bestandteils des biologischen Fluids zu entfernen.

5. Filtrationseinheit nach einem der Ansprüche 1 bis 4, **dadurch gekennzeichnet, dass** die Partikel (8) fähig sind, durch Affinität an mindestens einen Infektionserreger, wie etwa einen Virus, eine Bakterie, einen Parasiten, einen Pilz und ein Prionprotein und/oder an eine exogene Inaktivierungssubstanz zu binden.

6. Filtrationseinheit nach einem der Ansprüche 1 bis 5, **dadurch gekennzeichnet, dass** die Partikel (8) zwischen den Schichten (7) angeordnet sind.

7. Filtrationseinheit nach einem der Ansprüche 1 bis 5, **dadurch gekennzeichnet, dass** die Partikel (8) an mindestens einer Schicht (11) befestigt sind.

8. Filtrationseinheit nach einem der Ansprüche 1 bis 7, **dadurch gekennzeichnet, dass** die Partikel (8) aus Harzkugeln mit einem Durchmesser im Bereich zwischen 20 und 150 µm gebildet sind.

9. Filtrationseinheit nach einem der Ansprüche 1 bis 8, **dadurch gekennzeichnet, dass** die Menge der Partikel (8) zwischen zwei Schichten (7) im Bereich zwischen 4 und 10 mg/cm² liegt.

10. Filtrationseinheit nach einem der Ansprüche 1 bis 9, **dadurch gekennzeichnet, dass** mindestens zwei Schichten (7) einzeln miteinander verschweißt sind, um die Partikel (8) zwischen den Schichten (7) zurückzuhalten.

11. Filtrationseinheit nach einem der Ansprüche 1 bis 10, **dadurch gekennzeichnet, dass** die Ränder von mindestens zwei Schichten (7) miteinander verschweißt sind, so dass die Partikel (8) zwischen den Schichten (7) eingeschlossen sind.

12. Filtrationseinheit nach einem der Ansprüche 1 bis 11, **dadurch gekennzeichnet, dass** das poröse Material der stromabwärtigen Rückhaltestruktur (9) vom Typ Vlies ist.

13. Filtrationseinheit nach einem der Ansprüche 1 bis 12, **dadurch gekennzeichnet, dass** der mittlere Porendurchmesser der Schicht (10) der stromabwärtigen Rückhaltestruktur (9) kleiner als 10 µm ist.

14. Filtrationseinheit nach einem der Ansprüche 1 bis 13, **dadurch gekennzeichnet, dass** das Filtrationsmedium (5) ferner einen Vor- und/oder Nachfilter (14,15) umfasst.

15. Beutelsystem (16) zum selektiven Entfernen einer Zielsubstanz aus einem biologischen Fluid, wie etwa Blut, oder eines Blutbestandteils, **dadurch gekennzeichnet, dass** es einen Beutel zum Sammeln des Filtrats (17) umfasst, wobei der Beutel mittels einer Schlauchleitung (18) und im Bereich einer Einlassöffung (19) mit einer Auslassöffnung (4) einer Filtrationseinheit (1) nach einem der Ansprüche 1 bis 14 verbunden ist.
